Europäisches Patentamt

European Patent Office

Office européen des brevets

(19)

(11) Veröffentlichungsnummer: **0 138 179**
**A2**

(12) # EUROPÄISCHE PATENTANMELDUNG

(21) Anmeldenummer: **84112022.3**

(22) Anmeldetag: **06.10.84**

(51) Int. Cl.⁴: **A 61 L 15/00**

(30) Priorität: **14.10.83 DE 3337443**

(43) Veröffentlichungstag der Anmeldung: **24.04.85**
**Patentblatt 85/17**

(84) Benannte Vertragsstaaten: **AT BE CH DE FR GB IT LI LU NL SE**

(71) Anmelder: **Chemiefaser Lenzing Aktiengesellschaft, A-4860 Lenzing (AT)**

(72) Erfinder: **Sustmann, Scarlet, Dr., Am Nachtigallenwäldchen 34, A-4060 Viersen 12 (AT)**
Erfinder: **Marini, Ingo Gerhard, Dr., Hauptstrasse 40, A-4860 Lenzing (AT)**

(54) **Den pH-Wert regulierende Materialien und ihre Herstellung.**

(57) Die Erfindung betrifft den pH-Wert regulierende Materialien, die dadurch gekennzeichnet sind, daß der sie bildende Stoff eine einheitliche acide, chemisch modifizierte Viskosefaser ist, und ihre Herstellung.

Henkelstraße 67

4ooo Düsseldorf, 11.1o.1983

HENKEL KGaA
ZR-FE/Patente

AvK/Dr.JG

P a t e n t a n m e l d u n g

D 6775 EP

Den pH-Wert regulierende Materialien und ihre Herstellung

---

Die Erfindung betrifft den pH-Wert regulierende Materialien, die dadurch gekennzeichnet sind, daß der sie bildende Stoff eine einheitliche azide, chemisch modifizierte Faser ist, und ihre Herstellung.

Absorbierende Materialien bestehen aus hydrophilen Faserstoffen, wie Baumwoll-Lintern, Viskose, Schäumen o.ä.

Sofern die entsprechenden Materialien in direkten Hautkontakt kommen, hat es sich als vorteilhaft erwiesen, die Materialien mit Zusätzen zu versehen, die den pH-Wert der sie umgebenden Haut positiv beeinflussen und dadurch Reizungen bzw. erhöhte Anfälligkeit gegenüber Erkrankungen verhindern sollen.

So wurden z.B. Materialien für Tampons vorgeschlagen (DT 309575), die mit Glycogen, Zucker, Döderlein-Bakterien, Östrogenen etc. imprägniert sind,u.a. also solchen Substanzen, aus denen letztlich durch Lactobacillus acidophilus Milchsäure gebildet wird. Diese Zusätze haben jedoch den Nachteil, daß gerade während der Menstruation, wenn der pH-Wert der Vagina im alkalischen Bereich liegt, diese Bakterien in ihrem Wachstum stark gehemmt sind,so daß die Erzeugung von Milchsäure unter diesen Bedingungen nicht in wünschenswertem Ausmaß stattfinden kann. Außerdem wurde auch die unmittelbare Beaufschlagung der Materialien mit Milchsäure, Citronensäure o.ä. vorgeschlagen, DT 309575 und US-PS 979499. Derartige Imprägnierungen bzw. Zusätze haben den weiteren Nachteil, daß aufgrund der hohen Pufferwirkung der Menstruationsflüssigkeit bei den mit einem Tampon anzubietenden geringen Säuremengen die Wirkung bereits bei geringem Blutanfall erschöpft ist.

Die DE-OS 32 36 768 beschreibt Tampons, die eine oder mehrere Substanzen enthalten, die während der Benutzung des Tampons einen pH-Wert zwischen 2,5 und 4,5 erzeugen und aufrechterhalten und damit das Wachstum von pathogenen Bakterien verhindern. Als derartige Substanzen werden monomere und/oder polymere physiologisch verträgliche Carbonsäuren, wie Citronensäure, Äpfelsäure, Weinsäure oder Milchsäure genannt. Nachteil derartiger Tampons ist ebenfalls, daß die hohe Pufferwirkung der Körperflüssigkeiten der acidifizierenden Wirkung der in den Tampon eingebrachten Säuren Grenzen setzt.

In der US-PS 3 187 747 werden absorbierende Faser-Materialien mit Ionen-Austausch-Eigenschaften beschrieben, deren Zusammensetzung durch ein Vielkomponentensystem von Polymeren gekennzeichnet ist: Polymerkomponenten mit faserbildenden Eigenschaften, beispielsweise Textilfasern, liegen neben anderen Polymeren mit acidifizierenden Eigenschaften, beispielsweise Carboxymethylcellulose, in Form sogenannter "Polymer-Legierungen" ("alloy") vor, die aus homogenen Lösungen geeigneter Polymerer erhalten werden können. Die Heterogenität der Ausgangs-Polymeren bedingt, daß die Verarbeitung der Polymer-Legierungen zu den gewünschten Hygienemitteln zusätzliche Verfahrensschritte, wie

0138179

HENKEL KGaA
ZR-FE/Patente

z.B. eine Lösungsmittel-Austauschtrocknung, erfordert, um ein Entquellen der Fasern zu bewirken und damit zu verhindern, daß sie während des Trocknungsvorganges aneinander kleben. Außerdem werden derartige Fasern häufig noch mit kationenaktiven Weichmachern und Gleitmitteln behandelt, um ihre Verarbeitungseigenschaften zu verbessern, was jedoch die acidifizierenden Eigenschaften des Fasermaterials vermindert.

Ein weiteres Verfahren, bei dem ein bekanntes Material, z.B. modifizierte Viskosefaser, mit einer Substanz versetzt wird, die bei Gebrauch zu einer Absenkung des pH-Wertes führt, wird in der DE-OS 27 09 132 beschrieben. Zellstoff-Fasern werden mit Monochloressigsäure zu Carboxymethylcellulose umgesetzt; die resultierenden Carboxymethylcellulose-Fasern weisen einen durchschnittlichen Substitutionsgrad von 0,4 bis 2,0 auf. Diese Veretherung wird an der "fertigen" Faser durchgeführt. Nachteilig ist, daß diese nachträgliche Veretherung zu für die genannten Zwecke nur schlecht geeigneten Fasern mit schleimiger Oberfläche führt. Außerdem enthalten aus derartigen Fasern hergestellte Materialien freie Carboxylgruppen nur an der Faseroberfläche, was dazu führt, daß die in den Körperflüssigkeiten vorliegenden kationischen Substanzen eine schnelle und erschöpfende Desaktivierung der Faseroberfläche herbeiführen.

Es wurde nun überraschend gefunden, daß die genannten Nachteile nicht beobachtet werden, wenn man Fasern einsetzt, die bereits von sich aus saure Eigenschaften besitzen, d.h. welche ohne weitere Zusätze und nicht nur auf ihrer Oberfläche einen niedrigen pH-Wert aufweisen.

Dementsprechend betrifft die vorliegende Erfindung den pH-Wert regulierende Materialien, die dadurch gekennzeichnet sind, daß der sie bildende Stoff eine einheitliche acide, chemisch modifizierte Faser ist, und ihre Herstellung.

Das Prinzip der erfindungsgemäßen Materialien besteht darin, durch **Acidifizierung von handelsüblicher, in** alkalisierter Form vorliegender Carboxyalkylcellulose ein modifiziertes Material mit einem Substitutionsgrad von 0,01 bis 0,3 und einem Carboxylgruppengehalt von ca. 2% zu erhalten, das sich in seinen Verarbeitungseigenschaften wie auch in den Verwendungseigenschaften, z.B. in der Saugkapazität, praktisch nicht von normaler, nicht-modifizierter Zellwolle unterscheidet.

Als Fasern für die Herstellung der erfindungsgemäßen pH-Wert regulierenden Materialien sind z.B. VISCOSORB[R]-Fasern ( Hersteller: Chemiefaser Lenzing AG ) verwendbar. Es sind jedoch auch andere Fasern geeignet, die die genannten Anforderungen erfüllen. Als geeignete Fasern werden Fasern aus solchen Polymeren verstanden, die vor der Herstellung der eigentlichen Faser an den Hydroxygruppen des Rohmaterials bis zu einem Substitutionsgrad zwischen 0,01 und 0,3 auf an sich bekanntem Wege verethert worden sind. Das führt dazu, daß die Carboxylgruppen nicht nur auf der Faseroberfläche liegen, sondern über den gesamten Faserquerschnitt verteilt sind.

Die Herstellung der erfindungsgemäßen pH-Wert regulierenden Materialien erfolgt dadurch, daß die Carboxylatgruppen auf dem Markt erhältlicher alkalisierter Carboxyalkylcellulose-Watten in die freie Säureform überführt werden. Besonders geeignet sind die Produkte VISCOSORB 1S[R] und VISCOSORB 1N[R] der Firma Chemiefaser LENZING. Es können jedoch auch andere Watten eingesetzt werden.

Zur Acidifizierung werden die Fasern bei Raumtemperatur mit wäßrigen Mineralsäuren behandelt. Bevorzugt verwendet wird wäßrige Salzsäure in Konzentrationen von 0,1 bis 1 %, z.B. 0,2% wäßrige Salzsäure. Die Behandlungszeit beträgt 30 Minuten.

Anschließend wird die überschüssige Säure mit vollentsalztem Wasser ausgewaschen und das acidifizierte Material nach Abpressen des Wassers bei ca. 100°C getrocknet.

Die so hergestellten Fasern, die für pH-Wert-regulierende Materialien geeignet sind, haben einen Carboxylgruppengehalt von 1,9 bis 2,7% und besitzen dadurch saure Eigenschaften. Der Faser-pH-Wert, bestimmt nach dem Extrapolationsverfahren DIN 54 275, liegt zwischen 3,0 und 4,0, bevorzugt zwischen 3,4 und 3,9. Je nach Herstellungsbedingungen ist dieser Faser-pH-Wert jedoch variabel.

Die beschriebenen Fasern weisen im Vergleich zu herkömmlicher, stark saugfähiger Watte eine Reihe von Vorteilen auf.

Infolge des niedrigen Substitutionsgrades nach Überführung der Carboxylatgruppe in die freie Säureform entspricht die Saugkapazität und Saugkraft der erfindungsgemäßen Materialien derjenigen von normaler nativer bzw. regenerierter Cellulose oder Baumwolle.

Da die Modifikation, d.h. die Veretherung bereits am Cellulose-Rohmaterial vor der Faserherstellung durchgeführt wird und sich dadurch die freien Carboxyl-

Patentanmeldung D6775EP

HENKEL KGaA
ZR-FE/Patente

gruppen über den gesamten Faserquerschnitt verteilen, haben die erfindungsgemäßen pH-Wert-regulierenden Materialien eine deutlich bessere Pufferkapazität als erst an der fertigen Faser modifizierte Materialien. Sie sind dadurch in der Lage, einen sauren pH-Wert nicht nur einzustellen, sondern auch aufrechtzuerhalten.

Durch ein aus zwei oder mehr Polymerkomponenten bestehendes Fasermaterial bedingte zusätzliche Verfahrensschritte, wie z.B. eine Lösungsmittelaustauschtrocknung, fallen nicht an; die modifizierten, den pH-Wert regulierenden Materialien zeigen die in den Beispielen aufgeführten Eigenschaften ohne den Zusatz weiterer Komponenten, wie Weichmacher oder Gleitmittel. Mit Zusätzen derartiger Komponenten verbundene Nachteile müssen nicht in Kauf genommen werden.

Die beschriebenen, erfindungsgemäßen Materialien eignen sich unter anderem zur Herstellung von Schweißpolstern oder Schuheinlagen.

Die Erfindung wird durch die nachstehenden Beispiele näher erläutert.

Beispiel 1

Herstellung von azider Watte und Bestimmung der Faser-pH-Werte

Die Herstellung der Watten für die erfindungsgemäßen pH-Wert-regulierenden Materialien erfolgte durch Überführung der Carboxylatgruppen von aus carboxyalkyliertem Zellstoff über das Viskoseverfahren hergestellter, in alkalisierter Form vorliegender Caboxyalkylcellulose, z.B. der auf dem Markt erhältlichen Produkte

Patentanmeldung D6775 EP

VISCOSORB 1S[R] und VISCOSORB 1N[R], in die freie Säureform. Dazu wurden 1 kg Fasern mit 20 l 0,2%iger Salzsäure 30 Minuten lang bei Raumtemperatur behandelt. Anschliessend wurde auf eine Feuchte, von 200% abgepreßt und so lange mit vollensalztem Wasser gewaschen, bis das Waschwasser neutral reagierte. Danach wurde wiederum auf eine Feuchte von ca. 200% abgepreßt und 4 Stunden im Umlufttrockenschrank bei 105°C getrocknet. Die Überprüfung des Faser-pH-Wertes nach dem Extrapolationsverfahren DIN 54 275 erbrachte die in der Tabelle 1 aufgelisteten Ergebnisse:

Tabelle 1

Ermittlung des Faser-pH-Wertes nach DIN 54 275

| Fasermaterial | Carboxylgruppen | Faser-pH-Wert |
|---|---|---|
| VISCOSORB 1N[R] | 1,9 % | 3,4 |
| VISCOSORB 1S[R] | 2,7 % | 3,0 |
| Normalviskose | 0,3 % | 6,5 |

Beispiel 2

Flüssigkeitsrückhaltevermögen mit Wasser und Blutserum als Testflüssigkeit

Die Bestimmung des Flüssigkeitsrückhaltevermögens der erfindungsgemäßen Materialien erfolgte mit Wasser nach DIN 53 814, mit Blutserum in entsprechender Weise, jedoch ohne Zusatz von Netzmitteln. Die Ergebnisse sind nachfolgender Tabelle 2 zu entnehmen.

Patentanmeldung D 6775 EP

0138179

HENKEL KGaA
ZR-FE/Patente

## Tabelle 2

Flüssigkeitsrückhaltevermögen nach DIN 53 814

| Watte (% Feuchte nach Konditionierung[a]) | Rückhaltevermögen von | |
|---|---|---|
| | Wasser (%) | Serum (%) |
| Danufil[R] - Höchst (14,3) | 65,2 | 70,1 |
| VISCOSORB 1N[R] (17,8) | 54,3 | 69,6 |
| VISCOSORB 1S[R] (16,9) | 53,3 | 70,9 |

[a] Konditionierung: 20°C, 65% rel. Luftfeuchte

Ergebnis:

Im Test mit Blutserum wurden zwischen den verschiedenen Materialien keine Unterschiede festgestellt, während bei Wasser als Testflüssigkeit die erfindungsgemäßen Materialien ein etwas niedrigeres Rückhaltevermögen aufweisen.

Beispiel 3

In-vitro-Test zur Beeinflussung des pH-Wertes von Blutserum durch die erfindungsgemäßen Materialien

Zu je 10 bis 30ml Blutserum (pH-Wert 8,2) wurden 3,0g verschiedener Watten gegeben. Die Watteproben wurden wie in DIN 54 275 beschrieben behandelt. Nach 60min. Einwirkzeit der Testflüssigkeit wurde der Überstand von der Probe abzentrifugiert und der pH-Wert mit einem handelsüblichen pH-Meter mit Glaselektrode be-

Patentanmeldung  D6775 EP

HENKEL KGaA
ZR-FE/Patente

stimmt. Der Einfluß von azider Watte auf den pH-Wert im Vergleich zu normaler Zellwolle ist nachstehender Tabelle 3 zu entnehmen.

Tabelle 3
Prüfung des pH-Wertes nach DIN 54 275

| Watte (3,0 g) (Titer/Stapellänge) Serum | Serum-pH-Wert bei | | |
|---|---|---|---|
| | 10ml Serum | 15ml Serum | 30ml |
| ohne Wattezusatz | 8,2 | 8,2 | 8,2 |
| normale Zellwolle (3,6 dtex/30mm) | 7,88 | 8,04 | 8,17 |
| VISCOSORB 1S[R] (3,3 dtex/40mm) | 4,09 | 4,30 | 4,96 |
| VISCOSORB 1N[R] (3,6 dtex/30 mm) D6775 | 4,30 | 4,50 | 5,44 |

Ergebnis:

Auch bei 30ml Blutserum auf 3g Watte kann die Alkalität des Serums neutralisiert und der pH-Wert nahe dem physiologischen Bereich gehalten werden.

P a t e n t a n s p r ü c h e

1. Den pH-Wert regulierende Materialien, dadurch gekennzeichnet, daß der sie bildende Stoff eine einheitliche acide, chemisch modifizierte Faser ist.

2. Den pH-Wert regulierende Materialien nach Anspruch 1, dadurch gekennzeichnet, daß die Faser eine modifizierte Viskosefaser ist.

3. Den pH-Wert regulierende Materialien nach Ansprüchen 1 und 2, dadurch gekennzeichnet, daß die Viskosefaser eine durch covalent an das Cellulosegerüst über beliebige Brücken gebundene Carboxygruppen, bevorzugt durch $-CH_2COOH$-Gruppierungen modifizierte Viskosefaser ist.

4. Den pH-Wert regulierende Materialien nach Ansprüchen 1 - 3, dadurch gekennzeichnet, daß der Grad der Substitution der Viskosefaser durch Carboxy-Gruppen 0,01 - 0,3 beträgt.

5. Den pH-Wert regulierende Magterialien nach Ansprüchen 1 - 4, dadurch gekennzeichnet, daß der Carboxylgruppengehalt der sie bildenden Faser 1,9 bis 2,7% ist.

6. Den pH-Wert regulierende Materialien nach Ansprüchen 1 - 5, dadurch gekennzeichnet, daß die Materialien einen pH-Wert von kleiner 6, bevorzugt 3,0 bis 4,0, besitzen und aufrechterhalten.

7. Verfahren zur Herstellung von den pH-Wert regulierenden Materialien nach Ansprüchen 1 - 6, dadurch gekennzeichnet, daß man

a) aus in alkalisierter Form vorliegender
Caraboxyalkylcellulose, bevorzugt Carboxymethylcellulose bestehende Wattefasern bei Raumtemperatur einer 30-minütigen Behandlung mit wäßriger
Mineralsäure unterwirft;

b) die überschüssige Säure mit vollentsalztem Wasser
auswäscht;

c) das Wasser abpreßt und

d) die so acidifizierten Wattefasern bei ungefähr
100°C trocknet.

8. Verfahren nach Anspruch 7, dadurch gekennzeichnet,
daß man wäßrige Salzsäure verwendet.

9. Verfahren nach Ansprüchen 7 und 8, dadurch gekennzeichnet, daß die Säurekonzentration 0,1 bis 1 %, bezogen auf die eingesetzte wäßrige Säure, bevorzugt
0,2 %, beträgt.